(19) **Europäisches Patentamt**
**European Patent Office**
**Office européen des brevets**

(11) **EP 3 052 641 B1**

(12) **EUROPEAN PATENT SPECIFICATION**

(45) Date of publication and mention
of the grant of the patent:
**24.06.2020 Bulletin 2020/26**

(51) Int Cl.:
*C12P 21/06* [(2006.01)]   *C07K 1/12* [(2006.01)]
*A23J 3/30* [(2006.01)]   *A23L 33/17* [(2016.01)]

(21) Application number: **14850828.6**

(86) International application number:
**PCT/KR2014/001059**

(22) Date of filing: **07.02.2014**

(87) International publication number:
**WO 2015/050294 (09.04.2015 Gazette 2015/14)**

(54) **MANUFACTURING METHOD FOR A HYDROLYSATE OF ANIMAL PROTEIN**

HERSTELLUNGSVERFAHREN FÜR EIN HYDROLYSAT AUS TIERPROTEINEN

PROCÉDÉ DE PRODUCTION D'UN HYDROLYSAT DE PROTÉINE ANIMALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.10.2013 KR 20130118518**

(43) Date of publication of application:
**10.08.2016 Bulletin 2016/32**

(73) Proprietor: **Innoway Co. Ltd.**
**Anyang-si, Gyeonggi-do 431-804 (KR)**

(72) Inventors:
 • **SUNWOO, Hoon Heui**
 **Sherwood Park, Alberta T8A5J4 (CA)**
 • **LEE, Ho Young**
 **Uiwang-si**
 **Gyeonggi-do 437-761 (KR)**

(74) Representative: **Ter Meer Steinmeister & Partner**
**Patentanwälte mbB**
**Nymphenburger Straße 4**
**80335 München (DE)**

(56) References cited:
**EP-B1- 1 227 736    WO-A1-02/078461
WO-A1-2012/121612    KR-A- 20100 021 293
KR-A- 20130 085 803    US-A- 5 356 637
US-A1- 2008 009 611    US-A1- 2009 029 005
US-A1- 2011 082 281    US-A1- 2011 263 505**

 • **JOSEPH THOMAS RYAN ET AL: "Bioactive
 Peptides from Muscle Sources: Meat and Fish",
 NUTRIENTS, vol. 3, no. 12, 31 August 2011
 (2011-08-31), pages 765-791, XP055324353, DOI:
 10.3390/nu3090765**
 • **KRISTINSSON H G ET AL: "FISH PROTEIN
 HYDROLYSATES: PRODUCTION,
 BIOCHEMICAL AND FUNCTIONAL
 PROPERTIES", CRITICAL REVIEWS IN FOOD
 SCIENCE AND NUTRITION, TAYLOR & FRANCIS,
 USA, vol. 40, no. 1, 1 January 2000 (2000-01-01),
 pages 43-81, XP008003069, ISSN: 1040-8398,
 DOI: 10.1080/10408690091189266**
 • **IZQUIERDO F J ET AL: "Effects of high pressure
 and microwave on pronase and
 alpha-chymotrypsin hydrolysis of
 beta-lactoglobulin", FOOD CHEMISTRY,
 ELSEVIER LTD, NL, vol. 92, no. 4, 1 October 2005
 (2005-10-01), pages 713-719, XP027770055, ISSN:
 0308-8146 [retrieved on 2005-10-01]**

**Description**

[Technical Field]

The present invention relates to a method for manufacturing a hydrolysate of animal protein contained in meat, and more particularly, to a method for manufacturing a hydrolysate of animal protein degraded by an enzymatic hydrolysis under a high pressure and improved in rate of absorption from the small intestine or storage stability, and a method for manufacturing the hydrolysate of animal protein with reliability in a short time.

[Background Art]

Proteins obtained from foods provide amino acids required for protein synthesis in vivo. In general, the recommended intake of proteins is in the range of 0.8 to 1.2 g per standard or adjusted weight, and the recommended intake of proteins for Koreans is about 1.0 g per standard weight. Preferably, as proteins supplied to the human body, high-quality proteins with high bioavailability are mainly used. When calorie limit is more severe, a protein intake condition is important. Regarding weight control, when body fat is consumed, loss of muscle protein also occurs, but if dietary intake of protein is adequate, it is possible to minimize the loss of muscle protein. Patients and the elderly and infirm are recommended to take in high-quality meat protein containing all of the essential amino acids required for the human body to improve immunity. However, it has been reported that the patients and the elderly and infirm who have been fighting against diseases such as cancer or diabetes lack the digestive ability and has a very low digestion-absorption rate due to their weakened physical condition as compared with normal persons. Meanwhile, it is reported that when meat grilled at a high temperature is taken in, heterocyclic amines generated on the grilled meat, polycyclic aromatic hydrocarbons generated from smoke, and N-nitro compounds (NOCs as mutagenic substances contained in the meat may be a problem, and intake of red meat of 100 to 200 g or more per day increases the rate of colon cancer by 12 to 24% (Sandhu et al 2001; Norta et al 2002). Further, according to recent studies, it is found that heme iron contained in meat increases formation of NOC in a healthy person.

Since it was known that additional intake of protein improves athletic ability and strength, various meat protein products has recently been released and the size of the market for the meat protein products has been gradually increased. However, most commercial products as protein supplements have been sold as being preserved in the form of meat, and, thus, they may cause an uncomfortable feeling and does not have a smooth texture when taken in. Meanwhile, other commercial products in the form of hydrolyzed liquid protein are expected to be improved in preference and absorption rate into the body, and, thus, it is deemed that they can be differentiated from conventional protein foods. Further, if a protein product containing protein in a fixed amount per packaging unit is taken in, it is possible to prevent problems such as reduction in use of carbohydrate and reduction in athletic ability caused by excessive consumption.

According to the study on digestion and absorption of a protein hydrolysate, when there was made a comparison between hydrolyzed protein and intact protein on 10 old men, the hydrolyzed protein is promoted to be digested and absorbed from the intestine and there was an increase in availability of amino acids and introduction of dietary amino acids into the skeletal muscle after intake, as compared with the intact protein (Ren Koopman et al., 2009). Further, protein hydrolysates containing low molecular peptides have been used as substances for supply of high nutrient or for treatment (Bhaskar et al., 2007), and may be used, for example, for preparing a dietary composition for children having severe allergies (Mahmoud 1994). Furthermore, peptides are easily absorbed into the body and thus have been the best source for supply of nitrogen in sports nutrition, and peptides of high biological value have been highly useful as general protein supplements of various dietary products (Sliet et al., 2005).

Typically, a protein hydrolysate can be manufactured by enzyme, acid, or alkaline hydrolysis, and the enzyme hydrolysis of them is most efficient in terms of industrial applicability. The enzyme hydrolysis refers to a method in which a protease is applied to animal protein such as meat protein to dissolve and decompose the protein. By this method, a dissolved substance in the form of peptides can be obtained. During the process, amino acids capable of improving taste and flavor of the meat are released. Therefore, meat protein hydrolysates have been used as functional substances such as taste and flavor enhancers in the food industry. Meanwhile, the acid or alkaline hydrolysis includes a heat treatment, and thus, protein is coagulated by heat and physiochemical quality characteristics of the protein are changed due to protein denaturation, and production yield is also reduced.

Recently, in the field of food, a high-pressure technique has been used not only for sterilization but also for non-thermal treatment during a food production process. For example, there has been attempted a technique that can be used for degrading a material of a functional food to dissolve or extract nutrients and specific ingredients of the food while minimizing destruction or loss thereof at a temperature of 50°C or less. In particular, by the high-pressure process, efficiency of hydrolysis and extraction is increased and color, taste and flavor, and nutrients of effective components can be maintained. Due to such advantages, it is possible to produce products differentiated in functional characteristic from products produced by a conventional heat treatment. Regarding a method for producing a meat protein hydrolysate with a protease under a high pressure, Korean Patent Laid-open Publication No. 10-2010-0021293 (Prior Art 1) describes a method for producing beef seasoning by a high pressure/enzyme-dissolution process under a pressure of 50 to 125 MPa at a temperature of 40 to 60°C for 12 to 24 hours with addition of an enzyme of 0.1 to 0.5 wt.%. Further, Korean Patent Laid-open Publication No. 10-2013-0085803 (Prior Art 2) describes a method for increasing a degree of hydrolysis of meat protein by using a high pressurizer with meat, such as beef or chicken breast, of 10 to 40 wt.% with respect to water and a protease of 0.05 to 4.0 wt.% with respect to the meat for a time of 4 to 48 hours under a pressure of 25 to 400 MPa at a temperature of 25 to 60°C for high-pressure process. However, Prior Art 1 relates to a method for producing seasoning with the rate of hydrolysis of about 50%, and thus, it is limited in supplying protein to children, patients, and old people. Further, according to Prior Art 2, in a protein hydrolysate, a hydrolysate having a molecular weight of 1 kDa or less accounts for about 75%, and, thus, it is limited in supplying protein to children, patients, and old people. Further, while the protein hydrolysate is manufactured or stored for a long time, it may have a noisome odor caused by rancidification, resulting in a problem in terms of storage stability.

Hydrolysates obtained from animal proteins (meat, fish, eggs and dairy) are described in US 2009/029005 A1, US 2011/263505 A1, US 2011/082281 A1, WO 2012/121612 A1 and KR 2013 0085803 A.

J. T. RYAN ET AL, "Bioactive Peptides from Muscle Sources: Meat and Fish", NUTRIENTS, (20110831), vol. 3, no. 12, pages 765 - 791, describes bioactive peptides identified in a range of foods, including plant, milk and muscle, e.g. beef, chicken, pork and fish muscle proteins.

H. G. Kristinsson and B. A. Rasco, "Fish Protein Hydrolysates: Production, Biochemical, and Functional Properties", Critical Reviews in Food Science and Nutrition, vol. 40, no. 1, (2000-01-01, pages 43-81 discusses the chemical and biochemical characteristics of hydrolyzed fish proteins. F. J. IZQUIERDO ET AL, "Effects of high pressure and microwave on pronase and alpha-chymotrypsin hydrolysis of beta-lactoglobulin", FOOD CHEMISTRY, ELSEVIER LTD, NL, vol. 92, no. 4, (20051001), pages 713 - 719, studies the effects of hydrostatic high pressure, microwave irradiation, and conventional heating on hydrolysis of bovine beta-lactoglubolin AB by pronase and alpha-chymotrypsin.

[Disclosure of Invention]

[Technical Problem]

The present invention is conceived under the circumstances described above. An object of the present invention is to provide a method for manufacturing a hydrolysate of animal protein which is useful as a substance for supplying protein to children, patients, and old people, and in which rancidification or growth of microorganisms does not occur during a manufacturing process or a process of storage for a long time.

[Solution to Problem]

The inventors of the present invention have found that when meat protein is hydrolyzed with a protease under a high pressure, if fat contained in the meat is removed in advance, the hydrolysis is accelerated to obtain a degraded protein hydrolysate and rancidification can be prevented during a manufacturing process or a process of storage for a long time, and the degraded protein hydrolysate is excellent in rate of absorption from the small intestine wall and thus have completed the present invention. Further, the inventors of the present invention have found that when animal protein contained in an egg white or animal protein contained in milk whey is hydrolyzed with a protease under a high pressure, a degraded protein hydrolysate can be obtained with a high yield and thus have completed the present invention.

The present invention provides a manufacturing method of a hydrolysate of animal protein as defined in claim 1 comprising: preparing meat slurry by dispersing ground meat in water in an amount of 100 to 300 parts by weight with respect to 100 parts by weight of meat and removing fat floating to a surface of the water; and manufacturing an enzymatic hydrolysis product by carrying out an enzymatic hydrolysis to the meat slurry with addition of a protease in an amount of 0.25 to 5 parts by weight with respect to 100 parts by weight of the meat under a pressure of 75 to 200 MPa at a temperature of 40 to 60°C for 5 to 50 hours. Herein, the manufacturing method of the hydrolysate of animal protein according to the present invention further comprises obtaining a supernatant containing a hydrolysate of meat protein by cooling the enzymatic hydrolysis product at a temperature at which the fat is coagulated and removing a precipitate layer containing the coagulated fat or insoluble meat protein by centrifugation. Further, preferably, the manufacturing method of the hydrolysate of animal protein according to the exemplary embodiment of the present invention may further comprise obtaining a filtrate by inactivating the protease contained in the supernatant and performing filtering with a filter medium. Furthermore, preferably, the manufacturing method of the hydrolysate of animal protein according to the exemplary embodiment of the present invention may further comprise solidifying the filtrate, concentrating the filtrate, or solidifying a concentrated filtrate.

[Advantageous Effects of Invention]

In the case of manufacturing a hydrolysate of animal protein by the manufacturing method according to the present invention, a degree of hydrolysis is very high, and since rancidification or growth of microorganisms is suppressed during the manufacturing process, reliability of product quality and energy cost are excellent in economic terms. Further, in a hydrolysate of animal protein obtained by the method according to the present invention, most of the hydrolyzed protein is degraded into 1 kDa or less, and, thus, it is excellent in rate of absorption from the small intestine. Furthermore, the hydrolysate of animal protein obtained by the method according to the present invention is very low in fat, and thus, even if it is stored for a long time, rancidification or growth of microorganisms is suppressed and denaturation of the product does not occur. Therefore, the hydrolysate of animal protein obtained by the method according to the present invention can be used as a substance of a health functional food in the form of beverage, patient food, baby food, capsules, or tablets.

[Brief Description of Drawings]

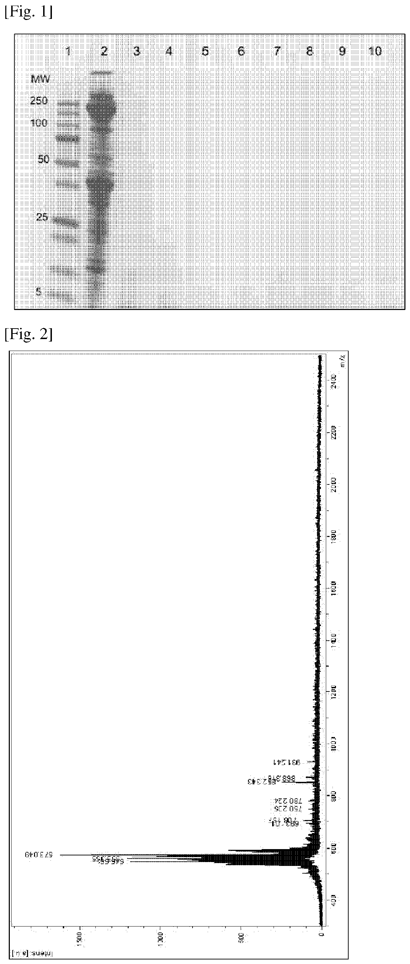

FIG. 1 illustrates a result of measurement on a molecular weight of a hydrolysate of meat protein by using electrophoresis. In FIG. 1, the first lane is an analysis result of a standard material; the second lane is an analysis result of protein contained in a round of beef before being enzymatically hydrolyzed; the third lane is an analysis result of a protein hydrolysate obtained by enzymatically hydrolyzing a round of beef for 4 hours; the fourth lane is an analysis result of a protein hydrolysate obtained by enzymatically hydrolyzing a chicken breast for 4 hours; the fifth lane is an analysis result of a protein hydrolysate obtained by enzymatically hydrolyzing pork tenderloin for 4 hours; the sixth lane is an analysis result of a protein hydrolysate obtained by enzymatically hydrolyzing a round of venison for 4 hours; the seventh lane is an analysis result of a protein hydrolysate obtained by enzymatically hydrolyzing a round of beef for 8 hours; the eighth lane is an analysis result of a protein hydrolysate obtained by enzymatically hydrolyzing a chicken breast for 8 hours; the ninth lane is an analysis result of a protein hydrolysate obtained by enzymatically hydrolyzing pork tenderloin for 8 hours; and the tenth lane is an analysis result of a protein hydrolysate obtained by enzymatically hydrolyzing round of venison for 8 hours.

FIG. 2 illustrates a result of a MALDI-TOF (Matrix Assisted Laser Desorption and Ionization-Time of Flight) mass spectrometry on a protein hydrolysate manufactured from a round of beef by a manufacturing method including a step of removing fat.

FIG. 3 illustrates a result of a MALDI-TOF (Matrix Assisted Laser Desorption and Ionization-Time of Flight) mass spectrometry on a protein hydrolysate manufactured from cow milk whey.

[Mode for the Invention]

**1. Manufacturing method of hydrolysate of animal protein according to the present invention**

A manufacturing method of a hydrolysate of animal protein according to the present invention uses meat as a source for supplying animal protein and includes a step for removing fat. Hereinafter, the manufacturing method of the hydrolysate of animal protein according to the present invention will be subdivided and explained.

Selection of meat

The term "meat" used in the present invention includes beef, pork, chicken, venison, salmon flesh, or tuna flesh. Further, preferably, the meat is relatively low in fat and relatively high in protein, and preferably, the meat may be selected from, for example, eye of round, tenderloin, or breast. To be specific, preferably, the meat used in the present invention may be selected from chicken breast, a round of beef, pork tenderloin, or a round of venison. Meanwhile, preferably, the fat contained in the meat may be adequately removed with a knife before the meat is ground.

Preparation of ground meat

The selected meat is ground into appropriate-sized pieces by a grinder. A size of the ground meat is not particularly limited, and considering easiness of handling, or improvement in efficiency of hydrolysis of a protease, a size of the ground meat is preferably 10 mesh or more, more preferably 20 mesh or more, and most preferably 30 mesh or more. For example, a size of the ground meat is 10 to 100 mesh, preferably 20 to 80 mesh, and more preferably 30 to 50 mesh. Meanwhile, in order to make it easy to grind the meat, a wet grinder may be used, and in this case, water may be added to the meat in an amount of 50 to 150 parts by weight, and preferably 50 to 100 parts by weight with respect to 100 parts by weight of the meat.

Manufacturing of meat slurry

After the prepared ground meat is dispersed in the water, fat floating to a surface of the water is removed and then meat slurry is manufactured. In this case, the water may be contained in the meat slurry in an amount of 100 to 300 parts by weight, and more preferably 150 to 250 parts by weight with respect to 100 parts by weight of the meat. Therefore, an amount of the water used when the ground meat is dispersed in the water is 100 to 300 parts by weight, and more preferably 150 to 250 parts by weight with respect to 100 parts by weight of the meat. Further, if the water is added to the meat when the meat is ground, an amount of the water used may be adjusted such that an amount of the water in the meat slurry may be in the range of 100 to 300 parts by weight with respect to 100 parts by weight of the meat. Meanwhile, since a part or most of the fat is removed when the meat slurry is manufactured, a fat content in the meat slurry is reduced to about 1/2 of the original meat. For example, a fat content in the meat slurry is about 1 to 2.5 % based on the total weight of the meat.

Manufacturing of enzymatic hydrolysis product

After the meat slurry is manufactured, a protease is added thereto, and the meat protein is hydro lyzed under a high pressure at a preset temperature for a preset period of time to manufacture an enzymatic hydrolysis product. The protease used herein includes one or more selected from the group consisting of Alcalase, Bromelain, Flavourzyme, $\alpha$-chymotrypsin, papain, trypsin acetylated, ficin, thermolysin, pancreatin, pepsin, trypsin, serine proteases, thereonine proteases, cysteine proteases, aspartate proteases, glutamic proteases, and metalloproteases, and may be preferably Alcalase considering economic feasibility.

Further, in the manufacturing method according to the exemplary embodiment of the present invention, an amount of the protease added is 0.25 to 5 parts by weight, more preferably 0.4 to 2 parts by weight considering a degree of hydrolysis of the meat protein and economic feasibility, and most preferably 0.4 to 1 part by weight with respect to 100 parts by weight of the meat. Furthermore, a pressure for the enzymatic hydrolysis is 75 to 200 MPa, more preferably 75 to 150 MPa considering a degree of hydrolysis of the meat protein and economic feasibility, and most preferably 80 to 120 MPa. Also, a temperature for the enzymatic hydrolysis is 40 to 60°C, more preferably 45 to 60°C considering a degree of hydrolysis of the meat protein and economic feasibility, and most preferably 45 to 55°C. Further, a time for the enzymatic hydrolysis is 5 to 50 hours, more preferably 5 to 24 hours considering a degree of hydrolysis of the meat protein and economic feasibility, and most preferably 6 to 12 hours.

Removal of fat from enzymatic hydrolysis product

The manufacturing method of the hydrolysate of animal protein according to the present invention further includes a step for removing fat from the enzymatic hydrolysis product. To be specific, a supernatant containing a hydrolysate of meat protein is obtained by cooling the enzymatic hydrolysis product at a temperature at which the fat is coagulated and removing a precipitate layer containing the coagulated fat or insoluble meat protein by centrifugation. In the enzymatic hydrolysis product, a small amount of fat is present besides the protein hydrolysate. If fat is present in a hydrolysate of meat protein, rancidification occurs, resulting in deterioration in product quality. In the manufacturing method of the hydrolysate of animal protein according to the exemplary embodiment of the present invention, before the centrifugation, the enzymatic hydrolysis product is cooled at a temperature at which the fat contained in the enzymatic hydrolysis product is coagulated. Herein, the temperature at which the fat is coagulated may be preferably 1 to 6°C, and more preferably 1 to 5°C. The temperature may be selected from various ranges depending on a kind or a distribution of fat present in the enzymatic hydrolysis product. In the manufacturing method according to the exemplary embodiment of the present invention, the enzymatic hydrolysis product manufactured by the enzymatic hydrolysis has a temperature of about 40 to 50°C. If the enzymatic hydrolysis product is centrifuged without cooling, the fat cannot be easily separated from the protein hydro lysate and may be present in the final hydro lysate of animal protein, resulting in deterioration in storage stability of the hydrolysate of animal protein. Further, in the manufacturing method according to the exemplary embodiment of the present invention, insoluble protein such as non-hydrolyzed protein is separated from water-soluble protein such as hydrolyzed protein by the centrifugation. In the manufacturing method of the hydrolysate of animal protein according to the exemplary embodiment of the present invention, most of the meat protein is hydrolyzed and converted into water-soluble protein, and, thus, insoluble protein in a very small amount is present in the enzymatic hydrolysis product. Meanwhile, if the enzymatic hydrolysis product is cooled at a temperature at which the fat is coagulated, and then centrifuged, a fat content in the supernatant containing the hydrolysate of meat protein is reduced to less than about 1% based on the total weight of solids (including the hydrolysate of meat protein). In the manufacturing method according to the exemplary embodiment of the present invention, the hydrolysate of meat protein in the supernatant obtained by the centrifugation contains hydrolyzed protein having a molecular size of 1 kDa or less at a ratio of 90% or more, preferably 95% or more, and more preferably 98 to 100%. Further, preferably, the hydrolyzed protein having a molecular size of 1 kDa or less has a molecular weight distribution of 100 Da or more to less than 1 kDa.

Inactivation of protease contained in supernatant and filtering

Preferably, the manufacturing method of the hydrolysate of animal protein according to the exemplary embodiment of the present invention may further include a step for inactivating the protease contained in the supernatant by performing a heat treatment on the supernatant obtained by the centrifugation under preset conditions and performing filtering with a filter medium. For example, the inactivation of the protease contained in the supernatant may include performing a heat treatment on the supernatant obtained by the centrifugation at 90 to 110°C for 5 to 30 minutes. Further, the filtering of the supernatant undergoing the inactivation of the protease may include allowing the supernatant to pass through a filter medium having a pore size of 0.1 to 100 $\mu$m, preferably 0.5 to 80 $\mu$m, and more preferably 10 to 60 $\mu$m so as to obtain a filtrate. Thereafter, the filtrate is sterilized by heating and then can be used as a liquid hydrolysate of meat protein.

Post-process of filtered liquid hydrolysate of meat protein

The liquid hydrolysate of meat protein obtained after the filtering can be converted into a liquid concentrate by various concentration methods such as vacuum evaporation. In the hydrolysate of meat protein in the form of liquid concentrate, a content of solids may be 60% or more, and preferably 80% or more. Further, the hydrolysate of meat protein in the form of liquid or the hydrolysate of meat protein in the form of liquid concentrate can be converted into the hydrolysate of meat protein in the form of powder by various solidification methods such as spray drying, or freeze drying.

## 2. Characteristic and use of hydrolysate of animal protein (reference embodiment)

The hydrolysate of animal protein is obtained by hydrolyzing animal protein with a protease in which hydrolyzed protein having a molecular size of 1 kDa or less accounts for 90% or more, preferably 95% or more, and more preferably 98 to 100% with respect to the total hydrolyzed protein. Further, the hydrolyzed protein having a molecular size of 1 kDa or less has a molecular weight distribution of preferably 100 Da or more to less than 1 kDa, and more preferably 400 to 900 Da. The hydrolysate of animal protein is mostly formed of degraded proteins (or peptides), and, thus, it is excellent in rate of absorption from the small intestine wall.

Animal protein used for manufacturing the hydrolysate of animal protein is not particularly limited as long as it is contained in meat, dairy products such as milk, eggs., and may be selected from, for example, meat protein, protein contained in an egg white, or milk whey protein. The meat protein refers to protein contained in edible meat of mammals and meat of fish and shellfish. The meat for supplying meat protein is not particularly limited in kind. For example, the meat may include one or more selected from the group consisting of beef, pork, chicken, venison, salmon flesh, or tuna flesh. Preferably, the meat may be selected from breast, eye of round, or tenderloin. Further, the egg white for supplying animal protein in the present invention is not particularly limited in kind as long as it is edible, and may be selected from the egg whites of various birds' eggs such as chicken eggs, ostrich eggs, or dug eggs. Furthermore, the milk whey for supplying animal protein in the present invention is not particularly limited in kind as long as it is edible, and may be selected from milk whey of milk from various mammals such as milk whey from cow milk, milk whey from mare milk, or milk whey from goat milk.

In the case of manufacturing the hydrolysate of animal protein from the meat, preferably, a fat content is limited to a preset level based on the total weight of solids in the hydrolysate of animal protein. For example, a content of fat contained in the hydrolysate of meat protein is preferably less than 1 wt.%, and more preferably less than 0.5 wt.% based on the total weight of the solids. The hydrolysate of animal protein according to the present invention is low in fat, and, thus, it is excellent in storage stability, and even if it is used as a substance of food, bad effects caused by intake of fat can be minimized.

The hydrolysate of meat protein can be used as a useful substance for supplying protein to children, patients, and old people. For example, the hydrolysate of meat protein in the form of liquid can be used as functional beverage. Further, the hydrolysate of meat protein in the form of liquid concentrate can be used as an additive of health functional foods such as beverages, patient foods, or baby foods. Furthermore, the hydrolysate of meat protein in the form of powder can be used as an additive of health functional foods in the form of beverage, patient food, baby food, and capsules, tablets.

The health functional food may include pills, powder, granules, infusions, tablets, capsules, or liquid medicines. To be specific, the food include, for example, meat, sausage, bread, chocolate, candies, snack foods, confectioneries, pizza, ramen, other noodles, gums, dairy products including ice creams, various soups, beverages, teas, functional water, health drinks, alcohol beverages, and vitamin complexes, and may include all of health functional foods in the accepted meaning. Further, the health functional food may contain various flavoring agents or natural carbohydrates as additional substances in addition to the hydrolysate of meat protein. Furthermore, the health functional food may contain various nutritional supplements, vitamins, electrolytes, flavoring agents, coloring agents, pectic acids and salts thereof, alginic acids and salts thereof, organic acids, protective colloid thickeners, pH conditioning agents, stabilizers, preservatives, glycerin, alcohols, or carbonation reagents used in carbonated drinks. In addition, the health functional food may contain fruit flesh for manufacturing natural fruit juices, fruit juice beverages, and vegetable beverages. Such components can be used independently or in combination. The natural carbohydrates include monosaccharides such as glucose and fructose, disaccharides such as maltose and sucrose, polysaccharides such as dextrin and cyclodextrin, and sugar-alcohols such as xylitol, sorbitol, or erythritol. As the flavoring agents, natural flavoring agents such as thaumatin or stevia extract or synthetic flavoring agents such as saccharine and aspartame may be used. Hereinafter, the present invention will be explained in more detail with reference to Examples. However, Examples below are provided just for clarifying a technical feature of the present invention but do not limit the protective scope of the present invention.

### 1. Selection of meat

As meat, chicken breast, a round of venison, pork tenderloin, and a round of beef were used. Table 1 below lists components of each meat used for manufacturing a hydrolysate of meat protein.

[Table 1]

| Meat | Content of component based on total weight of meat (wt.%) | | | |
|---|---|---|---|---|
| | Water | Crude protein | Crude fat | Crude ash |
| Chicken breast | 72.4 | 24.3 | 2.7 | 0.6 |
| Round of venison | 70.4 | 25.6 | 3.2 | 0.8 |
| Pork tenderloin | 70.8 | 24.6 | 3.7 | 0.9 |
| Round of beef | 73.4 | 20.1 | 5.8 | 0.7 |

### 2. Manufacturing of hydrolysate of meat protein and establishing optimal manufacturing conditions

Example 1: Degree of hydrolysis of meat protein depending on ratio of meat and purified water

Ground meat was prepared by grinding meat to have a size of about 30 to 60 mesh by a wet grinder. Then, the ground meat was dispersed in water in amount of 50 to 300 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 8 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 $\mu$m, and then, a filtrate was sterilized by heating at 121°C, thereby manufacturing a final hydrolysate of the meat protein.
Table 2 below lists a degree of a hydrolysis of meat protein depending on an amount of water used with respect to meat. Herein, the degree of hydrolysis of meat protein was obtained by cooling an enzymatic hydrolysis product, separating the enzymatic hydrolysis product into a water-soluble layer and a precipitate layer by centrifugation, analyzing a total amount of nitrogen contained in the contents of each layer by the Kjeldahl method, and making a calculation according to the following equation. In Examples provided hereinafter, a degree of hydrolysis of meat protein was obtained by the same method.
Degree of hydrolysis of meat protein (%) = [Total amount of nitrogen in water-soluble layer/ (Total amount of nitrogen in water-soluble layer + Total amount of nitrogen in precipitate layer)] x 100

[Table 2]

| Amount of purified water used with respect to 100 parts by weight of meat | Degree of hydrolysis of protein of each meat used (%) | | | |
|---|---|---|---|---|
| | Chicken breast | Round of beef | Pork tenderloin | Round of venison |
| 50 parts by weight | 82.3 | 79.5 | 78.5 | 78.6 |
| 100 parts by weight | 90.3 | 89.5 | 86.7 | 89.9 |
| 200 parts by weight | 99.5 | 99.5 | 99.5 | 99.5 |
| 300 parts by weight | 99.5 | 99.5 | 99.5 | 99.5 |

As can be seen from Table 2 above, when a hydrolysate of meat protein was manufactured, an optimal amount of water used was 100 to 300 parts by weight, and preferably 200 parts by weight with respect to 100 parts by weight of meat regardless of a kind of meat.

Example 2: Degree of hydrolysis of meat protein depending on amount of protease added

Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.1 to 5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 8 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 $\mu$m, and then, a filtrate was sterilized by heating at 121°C, thereby manufacturing a final hydrolysate of the meat protein.
Table 3 below lists a degree of a hydrolysis of meat protein depending on an amount of Alcalase 2.4 L added as a protease with respect to meat.

[Table 3]

| Amount of protease added with respect to 100 parts by weight of meat | Degree of hydrolysis of protein of each meat used (%) | | | |
|---|---|---|---|---|
| | Chicken breast | Round of beef | Pork tenderloin | Round of venison |
| 0.1 | 75.3 | 70.2 | 69.5 | 73.7 |
| 0.2 | 85.1 | 82.1 | 81.6 | 86.2 |
| 0.5 | 99.5 | 99.5 | 99.5 | 99.5 |
| 1 | 99.5 | 99.5 | 99.5 | 99.5 |
| 5 | 99.5 | 99.5 | 99.5 | 99.5 |

As can be seen from Table 3 above, when a hydrolysate of meat protein was manufactured, an optimal amount of a protease used was more than 0.2 parts by weight to 5 parts by weight, and preferably 0.5 parts by weight with respect to 100 parts by weight of meat regardless of a kind of meat.

Example 3: Degree of hydrolysis of meat protein depending on pressure for enzymatic hydrolysis

Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 25 to 200 MPa at a temperature of 50°C for 8 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 μm, and then, a filtrate was sterilized by heating at 121°C, thereby manufacturing a final hydrolysate of the meat protein.

Further, for comparison with a high pressure reaction, an enzymatic hydrolysis was carried out by the same method under atmospheric pressure and a final hydrolysate of the meat protein was manufactured.

Table 4 below lists a degree of a hydrolysis of meat protein depending on a pressure for an enzymatic hydrolysis.

[Table 4]

| Pressure (MPa) for enzymatic hydrolysis | Degree of hydrolysis of protein of each meat used (%) | | | |
|---|---|---|---|---|
| | Chicken breast | Round of beef | Pork tenderloin | Round of venison |
| 0.1 (Atmospheric pressure) | 45.1 | 49.4 | 45.4 | 47.2 |
| 25 | 65.3 | 69.2 | 70.8 | 68.7 |
| 50 | 85.5 | 82.8 | 84.2 | 82.8 |
| 75 | 97.5 | 95.3 | 93.5 | 96.7 |
| 100 | 99.5 | 99.5 | 99.5 | 99.5 |
| 200 | 92.8 | 90.3 | 93.5 | 95.8 |

As can be seen from Table 4 above, when a hydrolysate of meat protein was manufactured, an optimal pressure for an enzymatic hydrolysis was 75 to 200 MPa, and preferably 100 MPa regardless of a kind of meat. Meanwhile, in the case of the enzymatic hydrolysis of meat under atmospheric pressure, it was difficult to suppress microbial contamination and growth, and about 8 hours after the enzymatic hydrolysis, a noisome odor was emitted.

Example 4: Degree of hydrolysis of meat protein depending on temperature for enzymatic hydrolysis

Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 20 to 60°C for 8 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 μm, and then, a filtrate was sterilized by heating at 121°C, thereby manufacturing a final hydrolysate of the meat protein.

Table 5 below lists a degree of a hydrolysis of meat protein depending on a temperature for an enzymatic hydrolysis.

[Table 5]

| Temperature (°C) for enzymatic hydrolysis | Degree of hydrolysis of protein of each meat used (%) | | | |
|---|---|---|---|---|
| | Chicken breast | Round of beef | Pork tenderloin | Round of venison |
| 20 | 52.3 | 49.5 | 48.2 | 47.5 |
| 30 | 82.6 | 78.5 | 79.5 | 78.7 |
| 40 | 88.9 | 87.5 | 86.8 | 90.2 |
| 50 | 99.5 | 99.5 | 99.5 | 99.5 |

(continued)

| Temperature (°C) for enzymatic hydrolysis | Degree of hydrolysis of protein of each meat used (%) | | | |
|---|---|---|---|---|
| | Chicken breast | Round of beef | Pork tenderloin | Round of venison |
| 60 | 93.5 | 92.5 | 91.8 | 92.5 |

As can be seen from Table 5 above, when a hydrolysate of meat protein was manufactured, an optimal temperature for an enzymatic hydrolysis was 40 to 60°C, and preferably 50°C regardless of a kind of meat.

Example 5: Degree of hydrolysis of meat protein depending on time for enzymatic hydrolysis

Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 2 to 48 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 $\mu$m, and then, a filtrate was sterilized by heating at 121°C, thereby manufacturing a final hydrolysate of the meat protein.

Table 6 below lists a degree of a hydrolysis of meat protein depending on a time for an enzymatic hydrolysis.

[Table 6]

| Time (hr) for enzymatic hydrolysis | Degree of hydrolysis of protein of each meat used (%) | | | |
|---|---|---|---|---|
| | Chicken breast | Round of beef | Pork tenderloin | Round of venison |
| 2 | 79.2 | 65.5 | 62.3 | 69.8 |
| 4 | 88.3 | 80.1 | 82.6 | 84.5 |
| 8 | 99.5 | 99.5 | 99.5 | 99.5 |
| 24 | 99.5 | 99.5 | 99.5 | 99.5 |
| 48 | 99.5 | 99.5 | 99.5 | 99.5 |

As can be seen from Table 6 above, when a hydrolysate of meat protein was manufactured, an optimal time for an enzymatic hydrolysis was more than 4 hours to 45 hours, and preferably 8 hours regardless of a kind of meat.

Example 6: Degree of hydrolysis of meat protein depending on kind of protease

Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was selected and adjusted in the range of 5.0 to 8.0 depending on a kind of a protease to be used, and instead of Alcalase 2.4 L (Supplier: Sigma-Aldrich, U.S.A.), other proteases were added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 8 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 $\mu$m, and then, a filtrate was sterilized by heating at 121°C, thereby manufacturing a final hydrolysate of the meat protein.

The proteases used in Example 6 include Bromelain extracted from pineapples, Collupulin MG extracted from papayas, Flavourzyme 500MG, α-chymotrypsin, papain, trypsin acetylated, ficin, thermolysin, pancreatin, pepsin, trypsin, serine proteases, thereonine proteases, cysteine proteases, aspartate proteases, glutamic proteases, and metalloproteases, and all of the proteases used were purchased from Sigma-Aldrich (U.S.A.). In Example 6, a degree of hydrolysis of meat protein depending on a kind of a protease was 98% or more, which was not much different from the case of using Alcalase 2.4 L.

Comparative Example 1: Manufacturing of hydrolysis of meat protein by method without step for removing fat

Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under atmospheric pressure and a pressure of 100 MPa at a temperature of 50°C for 8 hours to manufacture an enzymatic hydrolysis product. Thereafter, the enzymatic hydrolysis product was cooled at 4°C, a precipitate layer containing non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 μm, and then, a filtrate was sterilized by heating at 121°C, thereby manufacturing a final hydrolysate of the meat protein.

Table 7 below lists a degree of a hydrolysis of protein of each meat used when a hydrolysate of meat protein was manufactured without a step for removing fat.

[Table 7]

| Pressure (MPa) for enzymatic hydrolysis | Degree of hydrolysis of protein of each meat used (%) | | | |
|---|---|---|---|---|
| | Chicken breast | Round of beef | Pork tenderloin | Round of venison |
| Atmospheric pressure (0.1) | 43.4 | 40.5 | 42.8 | 44.1 |
| 100 | 76.5 | 69.5 | 70.9 | 73.1 |

As can be seen from Table 7 above, when the enzymatic hydrolysis was carried out under atmospheric pressure, a degree of a hydrolysate of protein had a similar value regardless of whether or not fat was removed. However, it was difficult to suppress microbial contamination and growth, and about 8 hours after the enzymatic hydrolysis, a noisome odor was emitted. Further, when the enzymatic hydrolysis was carried out under a high pressure of 100 MPa, a hydrolysate of meat protein manufactured by a method without a step for removing fat had a degree of a hydrolysis of protein which was lower by about 25% than a hydrolysate of meat protein manufactured by a method including a step for removing fat.

## 3. Property analysis on hydrolysate of meat protein

(1) Fat content analysis in each step for manufacturing hydrolysate of meat protein In the method described in Example 5, 8 hours was selected as a time for an enzymatic hydrolysis to manufacture a final hydrolysate of meat protein. Further, as meat for manufacturing a hydrolysate of the meat protein, salmon flesh was added.

A fat content in each step for manufacturing the hydrolysate of meat protein was analyzed from meat before being ground, meat slurry from which fat was primarily removed after being ground, and a hydrolysate of meat protein from which fat was secondarily removed by cooling and centrifugation after an enzymatic hydrolysis. Table 8 below lists a result of the fat content analysis in each step for manufacturing the hydrolysate of meat protein.

[Table 8]

| Target of analysis | Fat content in meat (including hydrolysate of meat protein (%) | | | | |
|---|---|---|---|---|---|
| | Chicken breast | Round of beef | Pork tenderloin | Round of venison | Salmon flesh |
| Meat before being ground | 2.7 | 5.8 | 3.7 | 3.2 | 4.5 |
| Meat slurry from which fat was primarily removed after being ground | 1.4 | 2.4 | 1.8 | 1.6 | 1.5 |

(continued)

| Target of analysis | Fat content in meat (including hydrolysate of meat protein (%) | | | | |
|---|---|---|---|---|---|
| | Chicken breast | Round of beef | Pork tenderloin | Round of venison | Salmon flesh |
| Hydrolysate of meat protein from which fat was secondarily removed by cooling and centrifugation after enzymatic hydrolysis | 0.4 | 0.5 | 0.4 | 0.3 | 0.2 |

(2) Molecular weight distribution measurement on hydrolysate of meat protein using electrophoresis

In the method described in Example 5, 4 hours and 8 hours were selected as a time for an enzymatic hydrolysis to manufacture a final hydrolysate of meat protein.

Then, a molecular weight distribution of the hydrolysate of meat protein was measured by using electrophoresis. The hydrolysate of meat protein was filtered with a 0.2 $\mu$m syringe filter, and a filtrate was used as a sample of analysis. Further, a standard material having a molecular size of 5 kDa to 250 kDa was used as a marker, and protein contained in a round of beef before being enzymatically hydrolyzed was used as a control. FIG. 1 illustrates a result of measurement on a molecular weight of a hydrolysate of meat protein by using electrophoresis. In FIG. 1, the first lane is an analysis result of a standard material; the second lane is an analysis result of protein contained in a round of beef before being enzymatically hydrolyzed; the third lane is an analysis result of a protein hydrolysate obtained by enzymatically hydrolyzing a round of beef for 4 hours; the fourth lane is an analysis result of a protein hydrolysate obtained by enzymatically hydrolyzing a chicken breast for 4 hours; the fifth lane is an analysis result of a protein hydrolysate obtained by enzymatically hydrolyzing pork tenderloin for 4 hours; the sixth lane is an analysis result of a protein hydrolysate obtained by enzymatically hydrolyzing a round of venison for 4 hours; the seventh lane is an analysis result of a protein hydrolysate obtained by enzymatically hydrolyzing a round of beef for 8 hours; the eighth lane is an analysis result of a protein hydrolysate obtained by enzymatically hydrolyzing a chicken breast for 8 hours; the ninth lane is an analysis result of a protein hydrolysate obtained by enzymatically hydrolyzing pork tenderloin for 8 hours; and the tenth lane is an analysis result of a protein hydrolysate obtained by enzymatically hydrolyzing round of venison for 8 hours. As can be seen from Table 1, the hydrolysate of meat protein was not fractionated at all by the electrophoresis as compared with the meat protein before being hydrolyzed, which means that it is difficult to measure a molecular weight and a molecular weight distribution of the degraded hydrolysate of meat protein by the electrophoresis.

(3) Molecular weight distribution measurement on hydrolysate of meat protein using MALDI-TOF

1) Manufacturing of hydrolysate of meat protein by method including step for removing fat In the method described in Example 5, 8 hours was selected as a time for an enzymatic hydrolysis to manufacture a final hydrolysate of meat protein.
2) Manufacturing of hydrolysate of meat protein by method without step for removing fat Further, as a control, a hydrolysate of meat protein was manufactured by a method without a step for removing fat in the same manner as the method of Comparative Example 1.
3) Then, a molecular weight distribution of a hydrolysate of meat protein was measured by using MALDI-TOF (Matrix Assisted Laser Desorption and Ionization-Time of Flight) mass spectrometry. FIG. 2 illustrates a result of a MALDI-TOF (Matrix Assisted Laser Desorption and Ionization-Time of Flight) mass spectrometry on a protein hydrolysate manufactured from a round of beef by a manufacturing method including a step of removing fat. As can be seen from FIG. 2, 99% or more of the protein hydrolysate manufactured from a round of beef by the method including the step for removing fat exhibited a molecular weight distribution of 100 Da to less than 1000 Da. Further, 99% or more of the protein hydrolysate manufactured by using chicken breast, pork tenderloin, and a round of venison as meat by the method including the step for removing fat exhibited a molecular weight distribution of less than 1 kDa.

Table 9 below lists a molecular weight distribution of a hydrolysate of meat protein depending on whether or not a step for removing fat was included.

[Table 9]

| Manufacturing method of protein hydrolysate | Content of component having molecular weight of less than 1 kDa in protein hydrolysate of each meat used | | | |
|---|---|---|---|---|
| | Chicken breast | Round of beef | Pork tenderloin | Round of venison |
| Including step for removing fat | 99% or more | 99% or more | 99% or more | 99% or more |
| Without step for removing fat | 45% | 42% | 43% | 41% |

As can be seen from Table 9 above, 99% or more the protein hydrolysate manufactured by the method including the step for removing fat exhibited a molecular weight distribution of less than 1 kDa regardless of a kind of meat used, whereas 50% or more of the protein hydrolysate manufactured by the method without the step for removing fat exhibited a molecular weight distribution of 1 kDa or more regardless of a kind of meat used. (4) Qualitative analysis on storage stability of hydrolysate of meat protein In the method described in Example 5, 8 hours was selected as a time for an enzymatic hydrolysis to manufacture a final hydrolysate of meat protein.

A hydrolysate of protein contained in a round of beef was manufactured by using a round of beef as meat and then sealed in a container and shut off from light at each temperature of 4°C, 20°C, 37°C, and 50°C for 12 months, and then, smells, colors, acidity, and whether or not microbial growth occurred were monitored. As a result thereof, any change was not observed at any temperature, and a protein hydrolysate manufactured by using chicken breast, pork tenderloin, and a round of venison as meat by the same method exhibited the same result.

(5) Quantitative analysis on storage stability of hydrolysate of meat protein

1) Manufacturing of hydrolysate of meat protein by method including step for removing fat In the method described in Example 5, 8 hours was selected as a time for an enzymatic hydrolysis to manufacture a final hydrolysate of meat protein.

2) Manufacturing of hydrolysate of meat protein by method without step for removing fat Further, as a control, a hydrolysate of meat protein was manufactured by a method without a step for removing fat in the same manner as the method of Comparative Example 1.

3) Then, a liquid hydrolysate of meat protein was sealed in a container and shut off from light at room temperature for 12 months, and then, a degree of fat oxidation was measured by using a TBARS assay kit (Thiobarbituric acid reactive substances assay kit). Table 10 below lists a degree of fat oxidation of a hydrolysate of meat protein stored for 12 months. Herein, the degree of fat oxidation is expressed by an amount of malondialdehyde (MDA) present in a sample of measurement.

[Table 10]

| Manufacturing method of protein hydrolysate | Degree of fat oxidation of protein hydrolysate of each meat used after storage of 12 months (MDA M) | | | |
|---|---|---|---|---|
| | Chicken breast | Round of beef | Pork tenderloin | Round of venison |
| Including step for removing fat | 0.01 | 0.01 | 0.01 | 0.05 |
| Without step for removing fat | 1,245 | 2,379 | 0.956 | 1.424 |

As can be seen from Table 10 above, in the protein hydrolysate manufactured by the method including the step for removing fat, fat oxidation caused by long-time storage hardly occurred regardless of a kind of meat used. Meanwhile, in the protein hydrolysates manufactured by the method without the step for removing fat, fat oxidation caused by long-time storage occurred regardless of a kind of meat used, and particularly in the protein hydrolysates manufactured from the chicken breast and the round of beef, severe fat oxidation occurred.

**4. Measurement on function of hydrolysate of meat protein**

(1) Antioxidant effect of hydrolysate of meat protein

In the method described in Example 5, 8 hours was selected as a time for an enzymatic hydrolysis to manufacture a final hydrolysate of meat protein. Then, a concentration of an antioxidant substance contained in the final hydrolysate of the meat protein was measured. A concentration of the antioxidant substance was measured by using an antioxidant assay kit (Model name: CS0790; Supplier: Sigma, U.S.A.). After the hydrolysate of meat protein was dispersed well in a solution of myoglobin and ABTS [2,2'-azino-bis(3-ethylbenzothiazoline-6-sulphonic acid)], absorbance at 405 nm was measured by using a spectrophotometer. The measured absorbance was compared with a standard curve using Trolox (6-hydroxy-2,5,7,8-tetramethylchroman-2-carboxylic acid) and vitamin C to calculate a content (mM) of an antioxidant substance. Table 11 below lists a concentration of an antioxidant substance contained in a hydrolysate of protein of each meat.

[Table 11]

| Concentration of antioxidant substance contained in protein hydrolysate of each meat used (mM) | | | |
|---|---|---|---|
| Chicken breast | Round of beef | Pork tenderloin | Round of venison |
| 1.21 | 1.15 | 0.96 | 1.25 |

As can be seen from Table 11 above, the hydrolysate of the protein contained in the round of beef contained the greatest amount of the antioxidant substance.

(2) Rate of absorption from small intestine wall depending on molecular weight of hydrolysate of meat protein

In the method described in Example 5, 2 hours was selected as a time for an enzymatic hydrolysis to manufacture a final hydrolysate of meat protein. Then, the hydrolysate of meat protein was allowed to pass through UF membranes (Manufacturer: Millipore Corp., Bedford, MA, U.S.A.) having cut-off values of 10 KDa, 3 kDa, and 1 kDa in sequence to manufacture fractions at respective molecular weights of the hydrolysate of meat protein of 10 KDa or more, 3 to 10 kDa, 1 to 3 kDa, and less than 1 kDa. Thereafter, a rate of absorption from the small intestine wall of a fraction at each molecular weight of the hydrolysate of meat protein was measured.
As a small intestinal cell, a human small intestinal cell Caco2 was used. As a cell culture plate, an insert culture system produced by BD-Science was used. After a small intestine wall was artificially manufactured by culturing the human small intestinal cell in the cell culture plate, the fractions at respective molecular weights of the hydrolysate of meat protein were added in the same amount. After 2 hours, a content of protein (calculated into a nitrogen content) remaining in the small intestine wall and a content of protein passing through the small intestine wall were measured to calculate a rate of absorption from the small intestine wall. Table 12 below lists a measurement result on a rate of absorption from the small intestine wall with respect to a fraction at each molecular weight of the hydrolysate of meat protein.

[Table 12]

| Molecular weight of fraction of hydrolysate of meat protein | Rate of absorption from small intestine wall with respect to fraction of protein hydrolysate of each meat used (%) | | | |
|---|---|---|---|---|
| | Chicken breast | Round of beef | Pork tenderloin | Round of venison |
| 10 KDa or more | 5.2 | 5.0 | 4.5 | 5 |
| 3 to 10 kDa | 22 | 23 | 23 | 26 |
| 1 to 3 kDa | 55 | 52 | 53 | 59 |
| Less than 1 kDa | 90 | 88 | 89 | 92 |

As can be seen from Table 12 above, when the hydrolysate of meat protein had a molecular weight of less than 1 kDa, a rate of absorption from the small intestine wall was high, and particularly, in the hydrolysate of meat protein manufactured from the round of venison, a rate of absorption from the small intestine wall was the highest.

**5. Manufacturing of protein hydrolysate from animal proteins other than meat by high-pressure enzymatic hydrolysis**

Example 7 (not according to the invention) : Manufacturing of protein hydrolysate from egg white

A liquid egg white containing protein of about 10% was prepared by removing a shell and the yolk from an egg. Then, a pH of the liquid egg white was adjusted to about 6.0, and Alcalase 2.4 L (Supplier: Sigma-Aldrich, U.S.A.) was added thereto in an amount of 0.5 parts by weight with respect to 100 parts by weight of protein contained in the liquid egg white. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 8 hours to manufacture an enzymatic hydrolysis product. Thereafter, the enzymatic hydrolysis product was treated at 95°C for 20 minutes to inactivate an enzyme contained in the enzymatic hydrolysis product, and the enzymatic hydrolysis product was filtered with a filter medium having a pore size of about 50 μm, and then, a filtrate was sterilized by heating at 121°C, thereby manufacturing a protein hydrolysate from the egg white.

Example 8 (not according to the invention) : Manufacturing of protein hydrolysate from cow milk whey

A liquid cow milk whey containing protein of about 10% was prepared. Then, a pH of the liquid cow milk whey was adjusted to about 6.0, and Alcalase 2.4 L (Supplier: Sigma-Aldrich, U.S.A.) was added thereto in an amount of 0.5 parts by weight with respect to 100 parts by weight of protein contained in the liquid cow milk whey. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 8 hours to manufacture an enzymatic hydrolysis product. Thereafter, the enzymatic hydrolysis product was treated at 95°C for 20 minutes to inactivate an enzyme contained in the enzymatic hydrolysis product, and the enzymatic hydrolysis product was filtered with a filter medium having a pore size of about 50 μm, and then, a filtrate was sterilized by heating at 121°C, thereby manufacturing a protein hydro lysate from the liquid cow milk whey.

A total amount of nitrogen in the protein hydrolysate manufactured from the egg white and the protein hydrolysate manufactured from the liquid cow milk whey was measured by the Kjeldahl method, and the measured total amount of nitrogen was converted into an amount of protein, and then, production yield of the protein hydrolysate was calculated according to the following equation. As a result thereof, the production yield of the protein hydrolysate manufactured from the egg white and the protein hydrolysate manufactured from the liquid cow milk whey was 99% or more.

$$\text{Production yield of protein hydrolysate (\%)} = (\text{Total amount of nitrogen in protein hydrolysate} \times 6.25/ \text{Amount of protein contained in source of protein hydrolysate}) \times 100$$

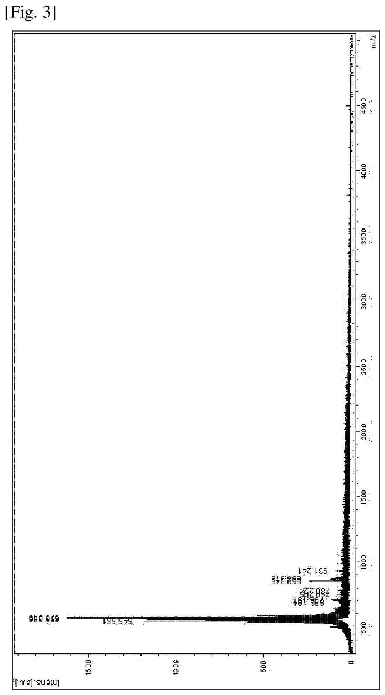

Further, a molecular weight distribution of the manufactured protein hydrolysate was measured by using MALDI-TOF (Matrix Assisted Laser Desorption and Ionization-Time of Flight) mass spectrometry. FIG. 3 illustrates a result of a MALDI-TOF (Matrix Assisted Laser Desorption and Ionization-Time of Flight) mass spectrometry on a protein hydrolysate manufactured from cow milk whey. 99% or more of the protein hydrolysates manufactured from the egg white and the cow milk whey by enzymatic hydrolysis under a high pressure exhibited a molecular weight distribution of less than 1 kDa.

**6. Various formulations of hydrolysate of meat protein and use thereof**

(1) Manufacturing of hydrolysate of meat protein in the form of liquid and use thereof In the method described in Example 5, 8 hours was selected as a time for an enzymatic hydrolysis to manufacture a final hydrolysate of meat protein. Since the final hydrolysate of meat protein is in the form of liquid, it can be used as functional beverage.

(2) Manufacturing of hydrolysate of meat protein in the form of liquid concentrate and use thereof Ground meat was prepared by grinding meat to have a size of about 30 to 60 mesh by a wet grinder. Then, the ground meat was dispersed in water in amount of 200 parts by weight with respect to 100 parts by weight of the meat and fat floating to a surface of the water was removed, thereby manufacturing meat slurry. Thereafter, a pH of the meat slurry was adjusted to 7.0, and Alcalase 2.4 L (Supplier: Sigma-Aldrich, U.S.A.) was added thereto in an amount of 0.5 parts by weight with respect to 100 parts by weight of the meat. Then, by using an ultrahigh pressurizer (Product name: TFS-10L; Manufacturer: Innoway Co., Ltd., Republic of Korea), an enzymatic hydrolysis was carried out under a pressure of 100 MPa at a temperature of 50°C for 8 hours to manufacture an enzymatic hydrolysis product. Thereafter, the enzymatic hydrolysis product was cooled at 4°C, a precipitate layer containing coagulated fat and non-hydrolyzed meat protein was removed by centrifugation (5,000 gf; 30 minutes), and a supernatant containing a hydrolysate of the meat protein was obtained. The supernatant was treated at 95°C for 20 minutes to inactivate an enzyme contained in the supernatant, and the supernatant was filtered with a filter medium having a pore size of about 50 μm, and then, a filtrate was vacuum-evaporated and sterilized at 50 to 100°C, thereby manufacturing a hydrolysate of the meat protein in the form of liquid concentrate. A content of solids in the hydrolysate of the meat protein in the form of liquid concentrate was 80% or more. The hydrolysate of the meat protein in the form of liquid concentrate can be used as an additive of health functional foods such as beverages, patient foods, or baby foods.

(3) Manufacturing of hydrolysate of meat protein in the form of powder and use thereof

In the method described in Example 5, 8 hours was selected as a time for an enzymatic hydrolysis to manufacture a hydrolysate of meat protein in the form of liquid. Then, the hydrolysate of meat protein in the form of liquid was freeze-dried to manufacture the hydrolysate of meat protein in the form of powder. The hydrolysate of meat protein in the form of powder can be used as an additive of health functional foods such as beverages, patient foods, baby foods, or capsules, tablets.

## Claims

1.  A manufacturing method of a hydrolysate of animal protein comprising:

    preparing meat slurry by dispersing ground meat in water in an amount of 100 to 300 parts by weight with respect to 100 parts by weight of meat and removing fat floating to a surface of the water; and
    manufacturing an enzymatic hydrolysis product by carrying out an enzymatic hydrolysis to the meat slurry with addition of a protease in an amount of 0.25 to 5 parts by weight with respect to 100 parts by weight of the meat under a pressure of 75 to 200 MPa at a temperature of 40 to 60°C for 5 to 50 hours; and
    obtaining a supernatant containing a hydrolysate of meat protein by cooling the enzymatic hydrolysis product at a temperature at which the fat is coagulated and removing a precipitate layer containing the coagulated fat or insoluble meat protein by centrifugation,
    wherein the meat includes one or more selected from the group consisting of beef, pork, chicken, venison, salmon flesh, and tuna flesh, and
    wherein the protease includes one or more selected from the group consisting of Alcalase, Bromelain, Flavourzyme, α-chymotrypsin, papain, trypsin acetylated, ficin, thermolysin, pancreatin, pepsin, trypsin, serine proteases, thereonine proteases, cysteine proteases, aspartate proteases, glutamic proteases, and metalloproteases.

2.  The manufacturing method of a hydrolysate of animal protein of claim 1, wherein the hydrolysate of meat protein contains fat of less than 1 wt.% based on the total weight of solids.

3.  The manufacturing method of a hydrolysate of animal protein of claim 1, wherein the temperature at which the fat is coagulated is 1 to 6°C.

4.  The manufacturing method of a hydrolysate of animal protein of claim 1, further comprising:
    obtaining a filtrate by inactivating the protease contained in the supernatant and performing filtering with a filter medium and optionally further comprising:
    solidifying the filtrate, or concentrating the filtrate or solidifying a concentrated filtrate.

5.  The manufacturing method of a hydrolysate of animal protein of claim 1, wherein the meat is selected from breast, eye of round, or tenderloin.

## Patentansprüche

1.  Verfahren zur Herstellung eines Hydrolysats von tierischem Protein, umfassend:

    Herstellen einer Fleischaufschlämmung durch Dispergieren von Hackfleisch in Wasser in einer Menge von 100 bis 300 Gewichtsteilen in Bezug auf 100 Gewichtsteile Fleisch und Entfernen von Fett, das zu einer Oberfläche des Wassers schwimmt; und

Herstellung eines enzymatischen Hydrolyseprodukts durch Durchführung einer enzymatischen Hydrolyse am Fleischbrei unter Zugabe einer Protease in einer Menge von 0,25 bis 5 Gewichtsteilen bezogen auf 100 Gewichtsteile des Fleisches unter einem Druck von 75 bis 200 MPa bei einer Temperatur von 40 bis 60°C für 5 bis 50 Stunden; und

Erhalten eines Überstandes, der ein Hydrolysat von Fleischprotein enthält, durch Abkühlen des enzymatischen Hydrolyseprodukts bei einer Temperatur, bei der das Fett koaguliert wird, und Entfernen einer Niederschlagsschicht, die das koagulierte Fett oder unlösliches Fleischprotein enthält, durch Zentrifugation,

wobei das Fleisch ein oder mehrere aus der Gruppe bestehend aus Rindfleisch, Schweinefleisch, Huhn, Wild, Lachsfleisch und Thunfischfleisch umfasst, und

wobei die Protease eine oder mehrere aus der Gruppe bestehend aus Alcalase, Bromelain, Flavourzym, α-Chymotrypsin, Papain, acetyliertes Trypsin, Ficin, Thermolysin, Pankreatin, Pepsin, Trypsin, Serinproteasen, Oninproteasen, Cysteinproteasen, Aspartatproteasen, Glutaminproteasen und Metalloproteasen umfasst.

**2.** Verfahren zur Herstellung eines Hydrolysats von tierischem Protein nach Anspruch 1, wobei das Hydrolysat von Fleischprotein weniger als 1 Gew.-% Fett, bezogen auf das Gesamtgewicht der Feststoffe, enthält.

**3.** Verfahren zur Herstellung eines Hydrolysats von tierischem Protein nach Anspruch 1, wobei die Temperatur, bei der das Fett koaguliert wird, 1 bis 6°C beträgt.

**4.** Verfahren zur Herstellung eines Hydrolysats von tierischem Protein nach Anspruch 1, ferner umfassend: Erhalten eines Filtrats durch Inaktivieren der im Überstand enthaltenen Protease und Durchführen des Filtrierens mit einem Filtermedium und gegebenenfalls ferner umfassend: Verfestigen des Filtrats oder Konzentrieren des Filtrats oder Verfestigen eines konzentrierten Filtrats.

**5.** Verfahren zur Herstellung eines Hydrolysats von tierischem Protein nach Anspruch 1, wobei das Fleisch aus Brust, Semerrolle oder Filet ausgewählt wird.

## Revendications

**1.** Procédé de fabrication d'un hydrolysat de protéines animales comprenant : la préparation d'une bouillie de viande en dispersant de la viande hachée dans de l'eau en une quantité de 100 à 300 parties en poids par rapport à 100 parties en poids de viande et en retirant la graisse flottant à la surface de l'eau;

et la fabrication d'un produit d'hydrolyse enzymatique en effectuant une hydrolyse enzymatique de la bouillie de viande avec l'addition d'une protéase en une quantité de 0. 25 à 5 parties en poids par rapport à 100 parties en poids de la viande sous une pression de 75 à 200 MPa à une température de 40 à 60°C pendant 5 à 50 heures;

et l'obtention d'un surnageant contenant un hydrolysat de protéine de viande en refroidissant le produit d'hydrolyse enzymatique à une température à laquelle la graisse est coagulée et en éliminant une couche de précipité contenant la graisse coagulée ou la protéine de viande insoluble par centrifugation, la viande comprenant un ou plusieurs éléments choisis dans le groupe constitué par le boeuf, le porc, le poulet, du gibier, de la chair de saumon et de la chair de thon,

et la protéase comprenant une ou plusieurs éléments choisis dans le groupe constitué par l'alcalase, la broméla ne, la flavourzyme, α-chymotrypsine, la papaïne, la trypsine acétylée, la ficine, la thermolysine, la pancréatine, la pepsine, la trypsine, les sérines protéases, les onyléines protéases, les cystéines protéases, les aspartate protéases, les glutamies protéases et les métalloprotéases.

**2.** Procédé de fabrication d'un hydrolysat de protéines animales selon la revendication 1, dans lequel l'hydrolysat de protéines de viande contient des matières grasses de moins de 1 % en poids par rapport au poids total des solides.

**3.** Procédé de fabrication d'un hydrolysat de protéines animales selon la revendication 1, dans lequel la température à laquelle la graisse est coagulée est de 1 à 6°C.

**4.** Procédé de fabrication d'un hydrolysat de protéines animales selon la revendication 1, comprenant en outre : l'obtention d'un filtrat par inactivation de la protéase contenue dans le surnageant et la réalisation d'une filtration avec un milieu filtrant et comprenant éventuellement en outre: la solidification du filtrat, ou la concentration du filtrat ou la solidification d'un filtrat concentré.

**5.** Procédé de fabrication d'un hydrolysat de protéines animales selon la revendication 1, dans lequel la viande est choisie parmi la poitrine, la tranche de noix ou le filet.

[Fig. 1]

[Fig. 2]

[Fig. 3]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020100021293
- KR 1020130085803
- US 2009029005 A1
- US 2011263505 A1
- US 2011082281 A1
- WO 2012121612 A1
- KR 20130085803 A

**Non-patent literature cited in the description**

- **J. T. RYAN et al.** Bioactive Peptides from Muscle Sources: Meat and Fish. *NUTRIENTS,* 31 August 2011, vol. 3 (12), 765-791
- **H. G. KRISTINSSON ; B. A. RASCO.** Fish Protein Hydrolysates: Production, Biochemical, and Functional Properties. *Critical Reviews in Food Science and Nutrition,* 01 January 2000, vol. 40 (1), 43-81
- Effects of high pressure and microwave on pronase and alpha-chymotrypsin hydrolysis of beta-lactoglobulin. **F. J. IZQUIERDO et al.** FOOD CHEMISTRY. ELSEVIER LTD, 01 October 2005, vol. 92, 713-719